# EUROPÄISCHE PATENTSCHRIFT

(11) **EP 3 073 955 B1**
(45) Veröffentlichungstag und Bekanntmachung des Hinweises auf die Patenterteilung: **07.10.2020**
(21) Anmeldenummer: 14799733.2
(22) Anmeldetag: 13.11.2014
(51) Int. Cl.: A61C 13/00, A61C 13/10, A61C 13/08, A61C 13/01

(54) **KÜNSTLICHER ZAHN SOWIE VERFAHREN ZUR HERSTELLUNG EINER PROTHESENBASIS**
ARTIFICIAL TOOTH AND METHOD FOR PRODUCING A PROSTHESIS BASE
DENT ARTIFICIELLE ET PROCÉDÉ DE FABRICATION D'UNE BASE DE PROTHÈSE

(30) Priorität: 26.11.2013 EP 13194419
(43) Veröffentlichungstag der Anmeldung: 05.10.2016
(73) Patentinhaber: Vita Zahnfabrik H. Rauter GmbH & Co. KG, 79713 Bad Säckingen (DE)
(72) Erfinder: FÖRTSCH, Jürgen, 79713 Bad Säckingen (DE); EGLE, Franz, 79713 Bad Säckingen (DE); KERSCHENSTEINER, Eva, 79713 Bad Säckingen (DE); CHRISTEN, Urban, 79713 Bad Säckingen (DE)
(74) Vertreter: dompatent von Kreisler Selting Werner - Partnerschaft von Patent- und Rechtsanwälten mbB
(86) Internationale Anmeldenummer: PCT/EP2014/074498
(87) Internationale Veröffentlichungsnummer: WO 2015/078701

(56) Entgegenhaltungen:
- EP-A1- 2 111 180
- WO-A1-2012/041329
- DE-A1- 3 925 324
- US-A- 5 938 442
- US-A1- 2013 108 988

## Beschreibung

Die Erfindung betrifft einen künstlichen Zahn zum Einsetzen in eine Prothesenbasis sowie ein Verfahren zur Herstellung einer Prothesenbasis.

Bei der Versorgung von Patienten mit dentalen Teil- oder Vollprothesen ist heutzutage ein hoher Standard festzustellen. Der Zahnarzt bereitet die zur Versorgung vorgesehene Situation am Patienten vor, während die Zahn(teil)prothese üblicherweise in einem externen Labor oder einem Praxislabor von einem Zahntechniker nach den Vorgaben des Zahnarztes hergestellt wird. Die Qualität der Prothese ist dabei in großem Maße abhängig von der handwerklichen Geschicklichkeit des Zahntechnikers, der die Vorgaben des Zahnarztes in der Herstellung der Prothese berücksichtigen muss, sowie der Qualität dieser Vorgaben.

Des Weiteren ist die Vorbereitung des prothetisch zu versorgenden Patienten für diesen oftmals mit Unannehmlichkeiten verbunden. Erwähnt seien in diesem Zusammenhang beispielsweise verschiedene Sitzungen beim behandelnden Zahnarzt, in denen Abformungen der zu versorgenden Situation vorgenommen werden müssen, die dem Zahntechniker als Negativmodelle dienen. Die sich anschließenden weiteren Abformungen sind zeitaufwendig und oftmals der Grund für fehlerhafte Ausführung der prothetischen Versorgung.

Typischerweise sind zwei oder drei Abformungssitzungen erforderlich, bevor der Zahntechniker mit der eigentlichen Herstellung der Prothese beginnen kann.

WO2011/066895 A1 betrifft ein Verfahren für die automatisierte Herstellung von Zahnersatz, umfassend die Schritte Bereitstellen eines digitalen Datensatzes der herzustellenden individuellen Prothese, digitale Trennung des Modells in Zahnbogen und Zahnfleisch, Herstellung des Zahnbogens aus Keramik und Kunststoff mittels Frästechnik oder Herstellung der Prothesenbasis durch generative oder abtragende Methoden aus überwiegend (Meth)-Acrylat-basierten Kunststoffen, den Anschluss des Zahnbogens und das Zahnfleisch durch Kleben oder Verbinden oder eine Kombination von Kleben und Fügen.

EP 2 111 180 A1 betrifft ein Verfahren zur Herstellung eines Basisteils eines künstlichen Gebisses oder eines künstlichen Gebisses, das ein Basisteil aufweist, mit dem Schritt Formen des Basisteils mit einem Rapidprototyping-Verfahren, wie z. B. der 3-D-Lithografie und insbesondere der 3-D-Laserlithografie. Weiterhin wird ein Verfahren zum Erstellen eines Datensatzes, der die Form eines Basisteils eines künstlichen Gebisses angibt offenbart, bei dem ein Zahnfleischareal oder ein Modell davon abgescannt wird und/oder ein Modell eines Basisteils abgescannt wird und/oder die Form des Basisteils auf einem Computer simuliert wird.

EP 1 864 627 A2 offenbart ein Verfahren zur Herstellung von Zahnprothesen nach einem digitalisierten, virtuellen Modell, welches die Kiefersituation wiedergibt, mit den Schritten einer digitalen Erfassung der Kiefersituation und -relationen, digitalen Aufstellung (automatisch als Option), Erzeugung einer geteilten Negativform (Rapid Manufacturing) aus den Daten der digitalen Zahnaufstellung, Einstecken der Konfektionszähne/ konfektionierten Zahneinheiten in die geöffneten Negativformen, Schließen der Negativformen, und Ausfüllen der verbliebenen Hohlräume mit Prothesenkunststoff.

EP 1 444 965 A2 betrifft ein Verfahren zur Herstellung von Zahnersatz, mit den Schritten, Aufnahme und Digitalisierung (Einscannen) der 3-dimensionalen, anatomischen Verhältnisse in der Mundhöhle, gegebenenfalls Aufnahme und Digitalisierung (Einscannen) der 3-dimensionalen Daten von Bissschablonen incl. Bisswällen, gegebenenfalls Aufnahme der Kieferdaten, die normalerweise am Patienten zur Einstellung des Artikulators genommen werden, Verarbeitung des erhaltenen Datensatzes in der Weise, dass die relevanten anatomischen Strukturen für eine virtuelle Zahnaufstellung festliegen und ein virtuelles Modell als Datensatz erhalten wird, gefolgt von der Auswahl der 3-D Datensätze konfektionierter, vorher eingescannter Zähne aus einem weiteren Datensatz, virtuelle Aufstellung der Zähne in das virtuelle Modell als zweiten Datensatz sowie entweder gefolgt von einem Übertragen der virtuellen Aufstellung auf das Modell durch entweder Positionierschablone (z.B. gefräst oder Rapid Prototyped) oder direkte Aufstellung der konfektionierten Zähne auf das Modell, Fixierung der Zähne auf dem Modell, Anbringen der Prothesenbasis oder in einer anderen Alternative, gefolgt von einer direkten Herstellung der Prothesenbasis - nach den Daten der virtuellen Zahnaufstellung - mit Positionierhilfen für die endgültige korrekte Positionierung und Fixierung der konfektionierten Zähne.

WO 2008/005432 A2 offenbart ein System zum Herstellen mindestens eines Teils einer Prothese. Das System umfasst eine dreidimensionale Abtastvorrichtung zum Abtasten einer Oberfläche einer Prothesenvorlage, und ein computerlesbares Medium mit einem Computerprogramm zum Empfangen von Daten aus der Abtasteinrichtung, einem 3-dimensionalen Modell der Oberfläche, und gegebenenfalls Modifizieren des 3-dimensionalen Modells und/oder das Hinzufügen von Funktionen auf das 3 - dimensionale Modell. Das System umfasst auch eine Verarbeitung zur Erzeugung mindestens eines Teils der Prothese aus einem ausgewählten Material, unter Berücksichtigung des 3-dimensionalen Modells. Die Verarbeitung kann materialabtragend oder aufbauend erfolgen.

Aus US 5,938,442 sowie WO 2012/041329 sind künstliche Zähne mit den Merkmalen des Oberbegriffs des Anspruchs 1 bekannt.

WO 2012/041329 offenbart dazu ein computergesteuertes Verfahren mit virtuellen Modellen zur Herstellung einer Prothesenbasis.

Aufgabe der Erfindung ist es zur Verbesserung des Herstellungsverfahrens einer Prothesenbasis geeignete künstliche Zähne bereitzustellen, wobei insbesondere ein automatisiertes Verfahren zur Herstellung einer Prothesenbasis ermöglicht werden soll.

Die Lösung der Aufgabe erfolgt durch einen künstlichen Zahn gemäß Anspruch 1, eine Prothese mit einer Prothesenbasis gemäß Anspruch 3 bzw. ein Verfahren zur Herstellung einer Prothesenbasis gemäß Anspruch 4.

Ein erfindungsgemäßer künstlicher Zahn zum Einsetzen in eine Prothesenbasis weist einen Außenteil und einen Innenteil auf. Der Außenteil ist derjenige Teil des Zahnes, der, wenn der Zahn in die Prothesenbasis eingesetzt ist, sichtbar bleibt. Der Innenteil ist der in eingesetztem Zustand des Zahns innerhalb der Prothesenbasis angeordnete Teil des Zahns. Erfindungsgemäß ist zwischen dem Außenteil und dem Innenteil eine Zahnkante ausgebildet. Das Ausbilden einer derartigen Zahnkante hat, wie nachstehend anhand des erfindungsgemäßen Verfahrens beschrieben, den Vorteil, dass diese Kante von einem automatischen Herstellungsverfahren wie einem CAD/CAM-System erkannt wird bzw. genutzt werden kann, um eine Kavität in der Prothesenbasis insbesondere automatisch bspw. durch Fräsen herzustellen. In diese Kavität wird sodann der künstliche Zahn in der Prothese eingesetzt, insbesondere eingeklebt.

Erfindungsgemäß erfolgt bei dem künstlichen Zahn an der Kante ein Winkelwechsel. Durch die Zahnkante ist somit eine klar erkennbare, definierte Kante bzw. Linie ausgebildet. Es erfolgt kein kontinuierlicher Übergang zwischen dem Außenteil und dem Innenteil des Zahns an der Zahnkante und eine sprunghafte Anderung des Winkels. Eine Funktion des Winkelverlaufs wäre somit im Bereich der Zahnkante unstetig. Der Winkelwechsel liegt bevorzugt im Bereich zwischen 3° - 80°. Der Winkel ergibt sich aus der Differenz der beiden angrenzenden Winkel.

Insbesondere aus fertigungstechnischen Gründen weist der künstliche Zahn keine mathematisch scharfe Kante auf. Vielmehr weist die Kante eine sehr geringe insbesondere fertigungsbedingte Rundung auf.

Erfindungsgemäß ist das Innenteil des künstlichen Zahns derart auszubilden, dass bezogen auf eine Einsetzrichtung bzw. Einschubrichtung, in der der Zahn in die Prothesenbasis eingesetzt wird, keine Hinterschnitte vorhanden sind. Hierdurch ist das Einsetzen erheblich vereinfacht. Insbesondere ist das Herstellen der Kavität in der Prothesenbasis, die dementsprechend auch keine Hinterschneidung in Einschubrichtung aufweist, deutlich einfacher möglich.

Der erfindungsgemäße künstliche Zahn ist erfindungsgemäß derart gebildet, dass die Zahnkante vollständig umlaufend ausgebildet ist. Es handelt sich bei der Zahnkante somit um eine in sich geschlossene Kante. Dies ist bei automatischen Herstellungsverfahren wie CAD/CAM-Verfahren vorteilhaft.

Des Weiteren ist es bevorzugt, dass der künstliche Zahn zur Individualisierung der Zahnform nur an dem Außenteil bearbeitet wird. Dies hat den Vorteil, dass ein insbesondere für die Automatisierung wichtiges, eindeutig definiertes Innenteil besteht und daher die Kavitäten in der Prothesenbasis automatisch hergestellt werden können. Die Form und die Tiefe der Kavitäten sind somit eindeutig vorgegeben.

Das Innenteil des künstlichen Zahns ist unsymmetrisch ausgebildet. Insbesondere ist das Innenteil nicht rotationssymmetrisch ausgebildet. Hierdurch ist ein Fehleinsetzen in der Prothesenbasis vermieden.

Zur vollständigen Herstellung einer Prothese ist eine Vielzahl von erfindungsgemäßen Zähnen in einer Bibliothek, aus der eine bestimmte Aufstellung an Zähnen ausgewählt werden kann vorhanden.

Ferner ist zur Herstellung einer Prothese eine Prothesenbasis vorgesehen. Die Prothesenbasis weist mehrere Kavitäten zur Aufnahme jeweils eines künstlichen Zahns, wie vorstehend beschrieben und vorteilhaft weitergebildet auf. Die einzelnen Kavitäten sind hierbei, bezogen auf eine Einsetzrichtung des entsprechenden künstlichen Zahns in die Prothesenbasis derart ausgebildet, dass die Kavitäten frei von Hinterschnitten sind. Hierdurch ist eine einfache Anordnung der künstlichen Zähne in der entsprechenden Prothesenbasis möglich.

Vorzugsweise sind die einzelnen Kavitäten der Prothesenbasis komplementär zu dem jeweiligen Innenteil des entsprechend einzusetzenden künstlichen Zahns ausgebildet. Hierdurch entsteht ein Spalt konstanter Breite zwischen der jeweiligen Kavität und dem Innenteil des zugehörigen Zahns. Hierdurch ist ein sicheres Befestigen des künstlichen Zahns in der Prothesenbasis gewährleistet.

Insbesondere bei der Verwendung von unsymmetrischen, insbesondere nicht rotationssymmetrisch ausgebildeten künstlichen Zähnen sind auch die Kavitäten entsprechend unsymmetrisch und insbesondere nicht rotationssymmetrisch in bevorzugter Ausführungsform ausgebildet. Dies hat den wesentlichen Vorteil, dass eine eindeutige Zuordnung zwischen dem jeweiligen künstlichen Zahn und der entsprechenden Kavität sowie auch eine eindeutige Lagedefinition des entsprechenden Zahns in der Kavität klar und eindeutig definiert ist.

Es ist daher besonders bevorzugt, dass alle Kavitäten eine unterschiedliche Form aufweisen, so dass eine eindeutige Zuordnung zwischen dem künstlichen Zahn und der Kavität besteht. Ein Fehleinsetzen ist somit ausgeschlossen.

Des Weiteren ist es bevorzugt, dass die Kavitäten derart ausgebildet sind, insbesondere eine derartige Tiefe aufweisen, dass ein eingesetzter künstlicher Zahn eine definierte Zahnhöhe hat.

Des Weiteren ist es insbesondere auch aus ästhetischen Gründen besonders bevorzugt, dass die Zahnkante des künstlichen Zahns, an der der Winkelwechsel erfolgt, zumindest teilweise insbesondere vollständig mit der Kavitätenkante im eingesetzten Zustand zusammenfällt bzw. sich die beiden Kanten, insbesondere vollständig überdecken.

Bei dem erfindungsgemäßen Verfahren zur Herstellung einer Prothesenbasis erfolgt ein virtuelles Einbetten mindestens eines erfindungsgemäßen künstlichen Zahns in eine virtuelle Prothesenbasis bzw. eine virtuelle Gingiva. Im nächsten Schritt wird auf Basis des Winkelwechsels an einem Zahnäquator der Zahnkante eine Kavitätenkante definiert. Dies erfolgt weiterhin virtuell, so dass für den mindestens einen erfindungsgemäßen Zahn in der virtuellen Prothesenbasis eine virtuelle Kavitätenkante erzeugt ist. Vorzugsweise ist die Kavitätenkante bei fertig gestellter Prothese mit eingesetztem künstlichen Zahn deckungsgleich mit der Zahnkante. Nach dem Definieren der mindestens einen Kavitätenkante erfolgt ein Herstellen der realen Prothesenbasis durch Einbringen einer Kavität in einen Prothesengrundkörper. Dies erfolgt insbesondere durch Ausfräsen der Kavität. Die Herstellung der Kavität erfolgt vorzugsweise durch ein CAD/CAM-Verfahren. Zur Durchführung von CAM-Verfahren dient erfindungsgemäß die Kavitätenkante zur Festlegung einer Werkzeugbewegung. Die Kavitätenkante dient hierbei als Grenzkurve für die Erstellung der Frässtrategie. Aus Grenzkurve und bekannter Kavitätengeometrie werden die Fräswege im CAM generiert. Für den Herstellungsprozess werden die, aus dem Stand der Technik bekannten, erforderlichen Parameter eines Fräsprozesses (Drehzahl, Vorschub, Zustellung seitlich und in der Tiefe,..) festgelegt.

Besonders bevorzugt ist es, dass das Definieren der virtuellen Kavitätenkante durch virtuelles Subtrahieren des Zahns von der virtuellen Prothesenbasis erfolgt. Hierdurch kann auf einfache Weise ein Übertragen der Zahnkante auf die virtuelle Prothesenbasis und somit ein Definieren der Kavitätenkante erzielt werden.

Die in der Prothesenbasis insbesondere durch CAD/CAM-Verfahren herzustellende Kavität ist in besonders bevorzugter Ausführungsform komplementär zur Unterseite bzw. zum Innenteil des Zahns ausgebildet. Hierdurch kann ein Klebespalt definiert werden, der über die gesamte Klebefläche eine konstante Breite bzw. Dicke aufweist. Dies hat den Vorteil, dass ein äußerst präzises und klar definiertes Einsetzen des Zahns in die Kavität gewährleistet ist. Des Weiteren besteht der Vorteil, dass aufgrund des klar definierten Klebespalts das Volumen des Klebespalts ebenfalls eindeutig definiert ist und insofern eine vorgegebene Menge an Klebstoff in die Kavität eingebracht werden kann, wobei ein Herausdrücken von Klebstoff im Bereich der Kavitäten- bzw. Zahnkante somit vermieden werden kann. Dies hat den Vorteil, dass die Nachbearbeitungsschritte vereinfacht werden oder ggf. vollständig entfallen können.

Insbesondere um eine gute Automatisierung zu ermöglichen ist es besonders bevorzugt, dass auch die Kavitäten in der Prothesenbasis derart ausgebildet sind, dass in Einsetzrichtung bzw. Einschubrichtung des Zahns keine Hinterschnitte auftreten.

Da die künstlichen Zähne vorzugsweise aus Kunststoff hergestellt werden und die Herstellung in üblicherweise zweiteiligen Formen erfolgt, entsteht eine Formteilkante. Die Formteilkante ist hierbei durch die Grenze der insbesondere zwei Formen definiert. Die Formteilkante muss hierbei derart angeordnet werden, dass ein einfaches Entformen des Zahns aus den Formteilen möglich ist. Die Formteile sind hierbei derart konstruiert, dass keine Hinterschnitte vorgesehen sind, um ein einfaches Herauslösen des Zahns aus dem Formteil zu gewährleisten. Besonders bevorzugt ist es, dass die Formteilkante zumindest teilweise mit der Zahnkante zusammenfällt. Dies hat den Vorteil, dass die Formteilkante zumindest teilweise in eingesetztem Zustand des Zahnes in die Prothesenbasis nicht sichtbar ist und insofern eine Nachbearbeitung nicht erforderlich ist.

Besonders bevorzugt ist es, dass mehrere insbesondere alle Zähne eines Zahnbogens anhand des erfindungsgemäßen vorstehend anhand eines Zahns beschriebenen Verfahrens virtuell in die virtuelle Prothesenbasis eingebettet werden. Entsprechend ist es bevorzugt, dass alle zugehörigen Kavitätenkanten insbesondere durch ein virtuelles Subtrahieren definiert werden und sodann wie vorstehend anhand eines Zahns beschrieben sämtliche zugehörigen Kavitäten vorzugsweise mit einem CAD/CAM-Verfahren hergestellt werden.

Vor dem erfindungsgemäßen Einbetten des mindestens einen erfindungsgemäßen Zahns in eine virtuelle Prothesenbasis können vorzugsweise folgende Verfahrensschritte durchgeführt werden:
- Erfassung einer oralen Patientensituation durch Abformung oder mittels digitaler Aufnahme,
- ggf. Digitalisierung der oralen Patientensituation,
- Auswahl der die dentale Prothese bildenden Zähne aus einer ideal errechneten Aufstellung nach entsprechendem Aufstellkonzept von Zähnen oder Zahngruppen, wobei eine virtuelle Aufstellung der Zähne erhalten wird, die virtuell in einem Raum positioniert ist, der die erfasste Patientensituation berücksichtigt,
- virtuelle Einbettung der in der virtuellen Aufstellung angeordneten Zähne in einer virtuellen Prothesenbasis.

Das Aufstellkonzept wird insbesondere patientenspezifisch, beispielsweise von einem behandelnden Arzt erarbeitet.

Unter Teil- oder Vollprothesen ist ein herausnehmbarer, bedingt herausnehmbarer oder festsitzender Zahnersatz zu verstehen.

Die Abformung der Situation im Mund des Patienten (Patientensituation) kann insbesondere durch Abformungsmassen erfolgen. Erfindungsgemäß lässt sich die Abformung vorteilhafterweise zur Erstabformung, Zweitabformung (Funktionsabformung) und Bissregistrierung nutzen.

Die Abformung lässt sich im erfindungsgemäßen Verfahren mit konventionellen Vorrichtungen, sogenannten Abformungslöffeln durchführen. Besonders geeignet ist ein Abformungslöffel, der eine schleimhautkongruente Auflage ermöglicht. Die Randbereiche sollen reduziert sein. Der Abformungslöffel soll zur Aufnahme von Abformungsmaterial dienen können und zur Aufnahme eines Vertikalmechanismus zur Bisseinstellung sowie posterioren Geometrien zur Bissfixierung fähig sein.

In einer Ausführungsform der vorliegenden Erfindung wird die digitale Aufnahme der Patientensituation mittels bildgebender, insbesondere optischer Verfahren wie Kameraaufnahme, Computertomographie, Ultraschall durchgeführt.

In einer weiteren Ausführungsform des erfindungsgemäßen Verfahrens enthält die Bibliothek Ausführungen von Zahnbögen, Zahnformen, Zahngrößen, und Kombinationen davon, wie sie sich beispielsweise aus den VITA Formenkarten Nr. 1694 und Nr. 1756 entnehmen lassen. Gesamtaufstellungen und/oder modulare Aufstellungen für alle Bissklassen nach Angle sind vorzugsweise in der Bibliothek berücksichtigt. Gewünschtenfalls kann die Bibliothek auch Ausgestaltungen der Gingiva in unterschiedlicher Altersausprägung enthalten.

Das erfindungsgemäße Verfahren ermöglicht vorteilhafterweise die Anpassung des Zahnbogens an die individuelle Kieferbogenweite des Patienten durch Berücksichtigung von virtuellen Scharnieren die durch eine Funktion in Form eines Bewegungselements (Bewegung nach innen und nach außen), beispielsweise zwischen Zahn 11 und 21 im Oberkiefer und Zahn 31 und 41 im Unterkiefer, erweitert werden. Die Ausgestaltung der Gingiva kann virtuell z.B. durch Einsetzen von Gaumenfalten oder Anpassung der A-Linie sowie der Radierungen individualisiert werden. Für die Gaumenfalten werden dabei vorzugsweise hinterlegte, virtuelle Modelle der Falten in das virtuelle Modell der Prothese eingesetzt. Hierbei kann es zweckmäßig sein, die Modelle entsprechend der Prothesengestalt individuell zu verändern, z.B. durch Streckung oder Stauchung sowie durch Mapping auf die Gaumenoberfläche. Die Radierung wird durch virtuelle Veränderung der Materialstärke in der Nähe der A-Linie modifiziert, die A-Linie selbst kann als Kurve bzw. Berandungslinie im virtuellen Modell angezeigt und verändert werden.

Diese Verfahrensschritte sind in PCT/EP2013/062279 detailliert beschrieben.

Nachfolgend wird die Erfindung anhand einer bevorzugten Ausführungsform unter Bezugnahme auf die anliegenden Zeichnungen näher erläutert.

Es zeigen:
- Fig. 1: eine schematische perspektivische Seitenansicht eines erfindungsgemäßen künstlichen Zahns,
- Fig. 2: eine schematische perspektivische Rückansicht eines erfindungsgemäßen künstlichen Zahns,
- Fig. 3: eine schematische perspektivische Draufsicht einer Kavität in einer Prothesenbasis,
- Fig. 4: eine schematische Schnittansicht eines in die Kavitätenbasis eingesetzten Zahn und
- Fig. 5: eine perspektivische Ansicht einer Prothesenbasis mit teilweise eingesetzten künstlichen Zähnen.

Zur Herstellung einer vollständigen Prothese, d.h. einer Prothesenbasis, in die künstliche Zähne eingesetzt sind, werden bspw. die folgenden Schritte durchgeführt:
- Erfassung einer oralen Patientensituation durch Abformung oder mittels digitaler Aufnahme,
- ggf. Digitalisierung der oralen Patientensituation,
- Auswahl der die dentale Prothese bildenden Zähne aus einer ideal errechneten Aufstellung nach entsprechendem Aufstellkonzept von Zähnen oder Zahngruppen, wobei eine virtuelle Aufstellung der Zähne erhalten wird, die virtuell in einem Raum positioniert ist, der die erfasste Patientensituation berücksichtigt,-virtuelle Einbettung der in der virtuellen Aufstellung angeordneten Zähne in einer virtuellen Gingiva, bzw. virtuellen Prothesenbasis,
- Herstellung der realen Prothese.

Dieses Verfahren ist insbesondere in PCT/EP2013/062279 beschrieben.

Dieses Verfahren ist erfindungsgemäß derart weitergebildet, dass erfindungsgemäß ausgestaltete künstliche Zähne verwendet werden. Eine entsprechende Bibliothek weist daher eine Vielzahl erfindungsgemäß ausgestalteter Zähne auf. Diese weisen unterschiedliche Zahngrößen, -formen etc. auf. Gemeinsam ist den erfindungsgemäßen künstlichen Zähnen insbesondere die Ausgestaltung einer Zahnkante mit Winkelwechsel.

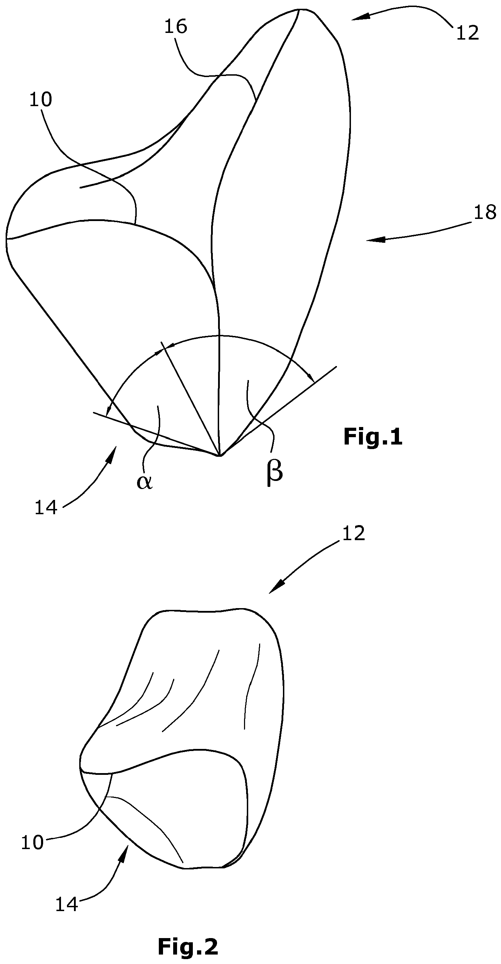

An dem in Fign. 1 und 2 dargestellten Beispiel eines Zahns, bei dem es sich um einen Schneidezahn handelt, ist die Zahnkante 10 sichtbar. Der als Beispiel dargestellte künstliche Zahn weist ein Außenteil 12 und ein Innenteil 14 auf.

Das Außenteil 12 ist von dem Innenteil 14 durch die Zahnkante 10 getrennt. Wenn der künstliche Zahn in die Prothesenbasis eingesetzt ist, ist der Außenteil außerhalb der Prothesenbasis angeordnet bzw. sichtbar. Der Innenteil 14 ist in eingesetztem Zustand innerhalb der Prothesenbasis angeordnet und insofern nicht sichtbar.

Die Zahnkante 10 ist als in sich geschlossene umlaufende Kante ausgebildet. An dieser Kante ist der erfindungsgemäße bevorzugte Winkelwechsel vorgesehen. Der Übergang zwischen dem Außenteil 12 und dem Innenteil 14 ist im Bereich der Zahnkante 10 somit nicht kontinuierlich oder stetig. Vielmehr weist der Winkel einen sprunghaften Wechsel auf. Der Winkel ergibt sich aus der Differenz der beiden angrenzenden Winkel α und β.

Da es sich bei dem künstlichen Zahn vorzugsweise um einen aus Kunststoff hergestellten Zahn handelt, ist dieser mit Hilfe von insbesondere zwei Formteilen hergestellt. Durch das Vorsehen von Formteilen entsteht eine Formkante 16. Diese ist vorzugsweise derart angeordnet, dass sie in einer Blickrichtung 18 nicht oder nur schwer sichtbar ist. Die Blickrichtung 18 stellt die Blickrichtung einer dritten Person auf die in den Mund des Patienten eingesetzte Prothese dar. Besonders bevorzugt ist es, dass die Formteilkante 16 zumindest teilweise mit der Zahnkante 10 zusammenfällt. Dies ist bezogen auf Fig. 1 in dem unteren Bereich der Fall, wobei die nicht dargestellte gegenüberliegende Seite des Zahns entsprechend ausgebildet ist.

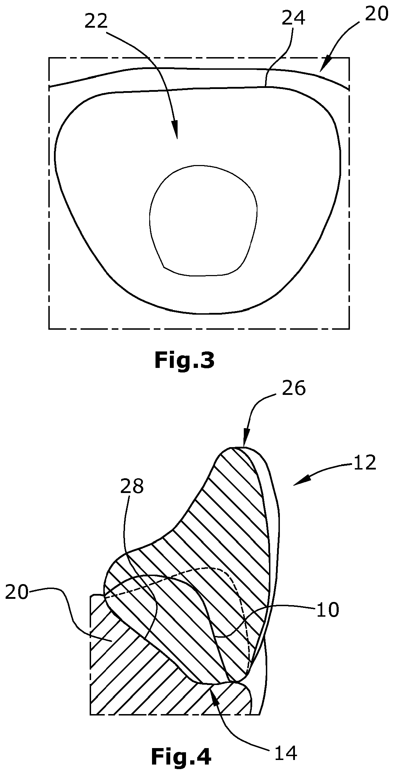

Zur Herstellung einer Prothesenbasis 20 (Fig. 3) erfolgt zunächst ein Subtrahieren der einzelnen Zähne von einer virtuellen Prothesenbasis. Virtuell kann somit eine Prothesenbasis 20 mit einer entsprechend für jeden Zahn ausgebildeten Kavität 22 erzeugt werden. Die virtuelle Prothesenbasis 20 weist somit eine Kavität 22 auf, die von einer Kavitätskante 24 begrenzt wird. Die Kavitätskante 24 wird durch die virtuelle Subtraktion des Zahns von der Prothesenbasis 20 erzeugt und entspricht der Zahnkante 10.

Die Herstellung der Kavitäten 22 in der Prothesenbasis 20 erfolgt vorzugsweise mit Hilfe von CAD/CAM-Verfahren. Diese nutzen die Kavitätenkante 24 zur Definition der Fräsbahnen bzw. der Bewegungsbahnen anderer Werkzeuge. Hierdurch ist eine sehr exakte und automatische Herstellung der Kavitäten 22 möglich.

Da es besonders bevorzugt ist, dass der Innenteil 14 der Zähne in eine Einsteckrichtung 26 (Fig. 4) der Zähne in die Kavitäten 22 keine Hinterschneidungen aufweist, ist eine einfache Montage bzw. ein einfaches Einsetzen/Einkleben in die Kavitäten 22 möglich. Die Kavitäten 22 weisen hierbei in die entsprechende Einsteckrichtung 26 ebenfalls keine Hinterschneidung auf. Die Einsteckrichtung 26 verläuft im Wesentlichen senkrecht zur Prothesenbasis. Bei in der Mundhöhle des Patienten angeordneter Prothese würde die Einstreckrichtung im Wesentlichen vertikal verlaufen.

Zur Vermeidung von Hinterschnitten sowohl am Innenteil 14 als auch in der Kavität 22 ist das Innenteil 14 ausgehend von der Zahnkante nach außen verjüngend ausgebildet. Insbesondere ist der Innenteil 14 zumindest teilweise konisch ausgestaltet. Die entsprechende Kavität 22 ist zu dem Innenteil komplementär ausgebildet, so dass eine exakte Lagedefinition des Zahns in der Kavität 22 gegeben ist. Durch eine komplementäre Ausgestaltung des Innenteils 14 und der Kavität 22 kann ferner ein Klebespalt 28 (Fig. 4) realisiert werden, der über seine gesamte Fläche eine konstante Breite aufweist. Insofern ist auch das Volumen des Klebespalts und somit die erforderliche Klebstoffmenge exakt bekannt.

In Fig. 4, in der der Zahn und auch die Prothesenbasis 20 geschnitten dargestellt sind, ist zusätzlich die Zahnkante 10 dargestellt. Hierbei verläuft der als durchgezogene Linie dargestellte Teil der Zahnkante 10 bezogen auf die Schnittfläche des Zahns vor der Zeichenebene und der gestrichelt dargestellte Teil hinter der Zeichenebene.

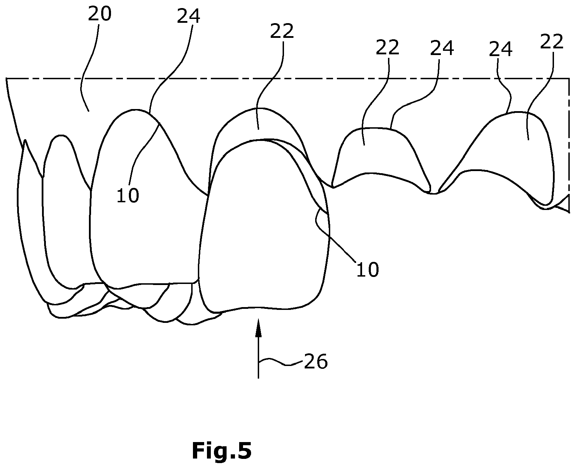

Zur vollständigen Herstellung einer Prothese sind in der Prothesenbasis 20 mehrere Kavitäten 22 vorgesehen (Fig.5). In die einzelnen Kavitäten wird zunächst eine klar definierte Menge Klebstoff eingebracht und sodann die entsprechenden Zähne in Einsteckrichtung 26 in die Kavitäten 22 eingesetzt. Bei eingesetzten Zähnen liegen die Kavitätenkante 24 und die Zahnkante 10 übereinander.

## Patentansprüche

1. Künstlicher Zahn zum Einsetzen in eine Prothesenbasis (20), mit
einem in eingesetztem Zustand sichtbaren Außenteil (12) und
einem in eingesetztem Zustand innerhalb der Prothesenbasis (20) angeordneten Innenteil (14),
wobei das Innenteil (14) bezogen auf eine Einsetzrichtung (26), in der der Zahn in die Prothesenbasis (20) eingesetzt wird, frei von Hinterschnitten ist und das Innenteil (14) unsymmetrisch, insbesondere nicht rotationssymmetrisch ausgebildet ist, und
wobei zwischen dem Außenteil (12) und dem Innenteil (14) eine Zahnkante (10) ausgebildet ist,
**dadurch gekennzeichnet, dass**
an der Zahnkante (10) ein sprunghafter Winkelwechsel erfolgt, so dass die Zahnkante (10) von einem automatischen Herstellungsverfahren erkennbar ist, um die Kavität in der Prothesenbasis automatisch herstellen zu können, wobei die Zahnkante (10) vollständig umlaufend ausgebildet ist.

2. Künstlicher Zahn nach Anspruch : 1, **dadurch gekennzeichnet, dass** eine Formteilkante (16) zumindest teilweise mit der Zahnkante (10) zusammenfällt.

3. Prothese mit einer Prothesenbasis, in die künstliche Zähne eingesetzt sind,
wobei die künstlichen Zähne einen in eingesetztem Zustand sichtbaren Außenteil (12) und einen in eingesetztem Zustand innerhalb der Prothesenbasis (20) angeordneten Innenteil (14) aufweisen, sodass zwischen dem Außenteil (12) und dem Innenteil (14) eine Zahnkante (10) ausgebildet ist, an der ein sprunghafter Winkelwechsel erfolgt und das Innenteil (14) bezogen auf eine Einsetzrichtung (26), in der der Zahn in die Prothesenbasis (20) eingesetzt wird, frei von Hinterschnitten ist, und
wobei die Prothesenbasis mehrere Kavitäten (22) zur Aufnahme jeweils eines künstlichen Zahns aufweist und die Kavitäten (22) bezogen auf eine Einsatzrichtung (26) in der der Zahn in die Prothesenbasis (20) eingesetzt wird, frei von Hinterschnitten sind,
wobei sich Kavitätenkanten (24) der Prothesenbasis und Zahnkanten (10) der künstlichen Zähne zumindest teilweise insbesondere vollständig überdecken und
wobei die Zahnkante (10) von einem automatischen Herstellungsverfahren erkennbar ist, um die Kavität in der Prothesenbasis automatisch herstellen zu können, und wobei die Zahnkante (10) vollständig umlaufend ausgebildet ist.

4. Verfahren zur Herstellung einer Prothesenbasis mit den Schritten:
virtuelles Einbetten mindestens eines Zahns nach einem der Ansprüche 1 bis 2 in eine virtuelle Prothesenbasis (20),
Definieren einer Kavitätenkante (24) auf Basis des Winkelwechsels an der Zahnkante (10),
Erkennen der Zahnkante (10) von einem automatischen Herstellungsverfahren und
Herstellen der reellen Prothesenbasis (20) durch Einbringen mindestens einer Kavität (22) in einen Prothesengrundkörper unter Nutzung der erkannten Zahnkante (10).

5. Verfahren zur Herstellung einer Prothesenbasis nach Anspruch 4, bei welchem die Kavitätenkante (24) durch virtuelles Subtrahieren des Zahns von der virtuellen Prothesenbasis (20) definiert wird.

6. Verfahren zur Herstellung einer Prothesenbasis nach Anspruch 4 oder 5, bei welchem das Herstellen der Kavität (22) durch CAD/CAM-Verfahren erfolgt, wobei die Kavitätenkante (24) zur Festlegung einer Werkzeugbewegung dient.

7. Verfahren zur Herstellung einer Prothesenbasis nach einem der Ansprüche 4 bis 6, bei welchem die Kavität (22) zur Ausbildung eines Klebespalts (28) konstanter Breite komplementär zum Innenteil (14) des Zahns gemäß einem der Ansprüche 1 bis 3 hergestellt wird.

8. Verfahren zur Herstellung einer Prothesenbasis nach einem der Ansprüche 4 bis 7, bei welchem die Kavitäten (22) in Einschubrichtung (26) des Zahns hinterschnittfrei hergestellt werden.

9. Verfahren zur Herstellung einer Prothesenbasis nach einem der Ansprüche 4 bis 8, bei welchem virtuell mehrere, insbesondere alle Zähne eines Zahnbogens eingebettet werden, alle zugehörigen Kavitätenkanten (24) definiert werden und alle zugehörigen Kavitäten (22) hergestellt werden.

10. Verfahren zur Herstellung einer Prothesenbasis nach einem der Ansprüche 4 bis 9, bei welchem vor dem virtuellen Einbetten des mindestens einen Zahns mindestens folgende Schritte durchgeführt werden:
Erfassung einer oralen Patientensituation durch Abformung oder mittels digitaler Aufnahme,
ggf. Digitalisierung der oralen Patientensituation,
Auswahl der die dentale Prothese bildenden Zähne aus einer ideal errechneten Aufstellung nach entsprechendem Aufstellkonzept von Zähnen oder Zahngruppen, wobei eine virtuelle Aufstellung der Zähne erhalten wird, die virtuell in einem Raum positioniert ist und zu der die erfasste Patientensituation passt,
virtuelle Einbettung der in der virtuellen Aufstellung angeordneten Zähne in einer virtuellen Prothesenbasis.

## Claims

1. Artificial tooth for insertion into a denture base (20), comprising
an outer part (12) visible in the inserted state, and
an inner part (14) arranged in the denture base (20) when in the inserted state,
wherein the inner part (14) is free of undercuts with respect to a direction of insertion (26) in which the tooth is inserted into the denture base (20), and the inner part (14) is asymmetric, in particular not rotationally symmetric in shape, and
wherein a tooth edge (10) is formed between the outer part (12) and the inner part (14),
**characterized in that**
an abrupt change of angle occurs at the tooth edge (10) so that the tooth edge (10) can be detected by an automatic production method to enable an automatic forming of a cavity in the denture base, wherein the tooth edge (10) is formed to extend all around the circumference thereof.

2. Artificial tooth of claim 1, **characterized in that** a mold parting edge (16) coincides at least in part with the tooth edge (10).

3. Denture comprising a denture base (20) in which artificial teeth are inserted, in which the artificial teeth comprise an outer part (12) visible in the inserted state and an inner part (14) arranged in the denture base (20) when in the inserted state, so that a tooth edge (10) is formed between the outer part (12) and the inner part (14) at which an abrupt change of angle occurs and the inner part (14) is free of undercuts with respect to a direction of insertion (26) in which the tooth is inserted into the denture base (20), and
wherein the denture base comprises a plurality of cavities (22) for receiving a respective artificial tooth, and wherein the cavities (22) are free of undercuts with respect to the direction of insertion (26) in which the tooth is inserted into the denture base (20),
wherein cavity edges (24) of the denture base and the tooth edges (10) of the artificial teeth overlap each other at least in part, in particular completely, and
wherein the tooth edge (10) can be detected by an automatic production method to enable an automatic forming of the cavity in the denture base, and wherein the tooth edge (10) is formed to extend all around the circumference thereof.

4. Method for producing a denture base, the method comprising the following steps:
virtual embedding of at least one tooth of one of claims 1 to 2 in a virtual denture base (20),
defining a cavity edge (24) based on the change of angle at the tooth edge (10),
detecting the tooth edge (10) by an automatic production method and
producing the real denture base (20) by forming at least one cavity (22) in a base body of a denture based on the tooth edge (10) detected.

5. Method for producing a denture base of claim 4, wherein the cavity edge (24) is defined by virtual subtraction of the tooth from the virtual denture base (20).

6. Method for producing a denture base of claim 4 or 5, wherein forming the cavity (22) is performed using a CAD/CAM process, the cavity edge (24) serving to define the tool movement.

7. Method for producing a denture base of one of claims 4 to 6, wherein the cavity (22) is produced to form a bonding gap (28) of constant width in a manner complementary to the inner part (14) of the tooth of one of claims 1 to 3.

8. Method for producing a denture base of one of claims 4 to 7, wherein the cavities (22) are formed without undercuts in the direction of insertion (26) of the tooth.

9. Method for producing a denture base of one of claims 4 to 8, wherein a plurality, preferably all teeth of a dental arch are embedded virtually, all associated cavity edges (24) are defined and all associated cavities (22) are formed.

10. Method for producing a denture base of one of claims 4 to 9, wherein at least the following steps are performed prior to the virtual embedding of the at least one tooth:
acquiring the oral situation of a patient by impressions or by digital recording,
if applicable, digitizing the oral situation of the patient,
selecting the teeth forming the denture from an ideal calculated set-up according to a corresponding set-up concept for teeth or groups of teeth, wherein a virtual set-up of the teeth is obtained that is positioned virtually in a space that takes into account the patient situation acquired,
virtual embedding of the teeth arranged in the virtual set-up into a virtual denture base.

## Revendications

1. Dent artificielle destinée à être insérée dans une base de prothèse (20), comprenant
une partie extérieure (12) visible à l'état inséré et
une partie intérieure (14) située, à l'état inséré, à l'intérieur de la base de prothèse (20),
par rapport à un sens d'insertion (26) dans laquelle la dent est insérée dans la base de prothèse (20), la partie intérieure (14) étant exempte de contre-dépouilles et présentant une forme asymétrique, notamment une forme non symétrique de révolution, et
un bord de dent (10) étant formé entre la partie extérieure (12) et la partie intérieure (14),
**caractérisée en ce que**,
au bord de dent (10), il y a un changement d'angle abrupt de façon que le bord de dent (10) soit reconnaissable par un procédé automatique de fabrication, afin de pouvoir réaliser de manière automatique la cavité dans la base de prothèse, le bord de dent (10) étant réalisé sur le pourtour entier.

2. Dent artificielle selon la revendication 1, **caractérisée en ce qu'**un bord de partie de moule (16) coïncide au moins partiellement avec le bord de dent (10).

3. Prothèse avec une base de prothèse dans laquelle des dents artificielles sont insérées,
les dents artificielles comprenant une partie extérieure (12) visible à l'état inséré et une partie intérieure (14) située, à l'état inséré, à l'intérieur de la base de prothèse (20), de façon qu'un bord de dent (10) soit formé entre la partie extérieure (12) et la partie intérieure (14) auquel il y a un changement d'angle abrupt, et par rapport à un sens d'insertion (26) dans laquelle la dent est insérée dans la base de prothèse (20), la partie intérieure (14) étant exempte de contre-dépouilles et
la base de prothèse comprenant plusieurs cavités (22) destinées à recevoir une dent artificielle respective, et par rapport à un sens d'insertion (26) dans laquelle la dent est insérée dans la base de prothèse (20), les cavités (22) étant exemptes de contre-dépouilles,
des bords de cavités (24) de la base de prothèse et des bords de dent (10) des dents artificielles se chevauchant au moins partiellement, notamment entièrement, et
le bord de dent (10) étant reconnaissable par un procédé automatique de fabrication, afin de pouvoir réaliser de manière automatique la cavité dans la base de prothèse, et le bord de dent (10) étant entièrement circonférentiel.

4. Procédé de fabrication d'une base de prothèse comprenant les étapes :
insertion virtuelle d'au moins une dent selon l'une des revendications 1 à 2 dans une base de prothèse (20) virtuelle,
définition d'un bord de cavité (24) sur la base du changement d'angle au bord de dent (10),
reconnaissance du bord de dent (10) par un procédé de fabrication automatique et fabrication de la base de prothèse (20) réelle par réalisation d'au moins une cavité (22) dans un corps de base de prothèse en utilisant le bord de dent (10) reconnu.

5. Procédé de fabrication d'une base de prothèse selon la revendication 4, dans lequel le bord de cavité (24) est défini par une soustraction virtuelle de la dent de la base de prothèse (20) virtuelle.

6. Procédé de fabrication d'une base de prothèse selon la revendication 4 ou 5, dans lequel la réalisation de la cavité (22) s'effectue selon des procédés CAD/CAM, le bord de cavité (24) servant à déterminer un mouvement d'un outil.

7. Procédé de fabrication d'une base de prothèse selon l'une des revendications 4 à 6, dans lequel, pour la formation d'une fente de collage (28) d'une largeur constante, la cavité (22) est fabriquée de façon complémentaire à la partie intérieure (14) de la dent selon une des revendications 1 à 3.

8. Procédé de fabrication d'une base de prothèse selon l'une des revendications 4 à 7, dans lequel les cavités (22) sont fabriquées, dans le sens d'insertion (26) de la dent, sans contre-dépouilles.

9. Procédé de fabrication d'une base de prothèse selon l'une des revendications 4 à 8, dans lequel plusieurs, en particulier toutes les dents d'une arcade dentaire sont insérées, tous les bords de cavité (24) afférents sont définis et toutes les cavités (22) afférentes sont fabriquées.

10. Procédé de fabrication d'une base de prothèse selon l'une des revendications 4 à 9, dans lequel au moins les étapes suivantes doivent être mises en œuvre avant l'insertion virtuelle de ladite au moins une dent :
enregistrement d'une situation orale du patient par moulage ou au moyen d'une empreinte numérique,
le cas échéant, numérisation de la situation orale du patient,
choix des dents formant la prothèse dentaire à partir d'une implantation calculée idéalement selon un concept correspondant d'implantation de dents ou de groupes de dents, une implantation virtuelle des dents étant obtenue qui est virtuellement positionnée dans un espace et à laquelle correspond la situation saisie du patient,
insertion virtuelle des dents disposées dans l'implantation virtuelle dans une base de prothèse virtuelle.
